# EUROPÄISCHE PATENTSCHRIFT

(11) **EP 0 665 830 B1**
(45) Veröffentlichungstag und Bekanntmachung des Hinweises auf die Patenterteilung: **12.03.1997**
(21) Anmeldenummer: 93924049.5
(22) Anmeldetag: 25.10.1993
(51) Int. Cl.: C07C 229/48, A61K 31/215

(54) **TILIDIN-DIHYDROGENORTHOPHOSPHAT, VERFAHREN ZU DESSEN HERSTELLUNG UND DIESES ENTHALTENDE PHARMAZEUTISCHE ZUBEREITUNGEN**
TILIDIN DIHYDROGEN ORTHOPHOSPHATE, METHOD OF PREPARING IT AND PHARMACEUTICAL PREPARATIONS CONTAINING IT
DIHYDROGENOORTHOPHOSPHATE DE TILIDINE, SON PROCEDE DE FABRICATION ET PREPARATIONS PHARMACEUTIQUES LE CONTENANT

(30) Priorität: 26.10.1992 DE 4236074
(43) Veröffentlichungstag der Anmeldung: 09.08.1995
(73) Patentinhaber: GÖDECKE AKTIENGESELLSCHAFT, D-10587 Berlin (DE)
(72) Erfinder: HERRMANN, Wolfgang, D-79104 Freiburg (DE); KNAPP, Armin, D-79279 Vörstetten (DE); KLAUSMANN, Hans, D-79244 Münstertal (DE); WITZKE, Joachim, Dr., 79331 Teningen-Nimburg (DE)
(74) Vertreter: Mansmann, Ivo
(86) Internationale Anmeldenummer: EP9302954
(87) Internationale Veröffentlichungsnummer: WO9410129

(56) Entgegenhaltungen:
- DE-B- 1 793 571
- CHEMICAL ABSTRACTS, vol. 83, no. 5, 1975, Columbus, Ohio, US; abstract no. 37590g, B. DUBINSKY ET AL. 'CORRELATION OF ANALGESIA WITH LEVELS OF TILIDINE AND A BIOLOGICALLY ACTIVE METABOLITE EN RAT PLASMA AND BRAIN' Seite 37584 ; & BIOCHEM. PHARAMACOL. Bd. 24, Nr. 2 , 1975 Seiten 277 - 281
- CHEMICAL ABSTRACTS, Bd. 83, Nr. 5, 1975, Columbus, Ohio, US; Zusammenfassung Nr. 37590g, B. DUBINSKY et al: "Correlation of analgesia with levels of tilidine and a biologically active metabolite in rat plasma and brain", Seite 37584

## Beschreibung

Tilidin, [(±)-Ethyl-(trans-2-dimethylamino-l-phenyl-3-cyclohexen-trans-1-carboxylat)] ist ein Analgetikum, das enteral rasch resorbiert wird und sich zur Behandlung sehr starker Schmerzen besonders eignet. Für die galenische Verarbeitung kommen vornehmlich Salze des basischen Wirkstoffs in Frage, da die Base als solche keine ausreichende Stabilität über einen längeren Zeitraum aufweist. Bisher ist es jedoch, ebenfalls aus Stabilitätsgründen, auch unter Verwendung von Salzen nicht gelungen, feste Arzneimittelformen, wie z.B. Tabletten oder Suppositorien zu entwickeln. Als Salz für stabile flüssige Zubereitungen hat sich in der Praxis bisher auch nur das Hydrochlorid-Semihydrat (DE-PS 1 518 959 und 1 793 571) als brauchbar erwiesen. Es ist in Form einer Lösung, bzw. einer in Weichgelatine-Kapseln portionierten Suspension unter dem Warenzeichen Valoron N im Handel. Aufgrund seiner überaus günstigen Eigenschaften bei der Schmerzbekämpfung hat sich Valoron N mittlerweile in Deutschland zu einem der führenden Analgetika entwickelt. Alle Anstrengungen, brauchbare feste Arzneimittelformen des Wirkstoffs Tilidin bereitzustellen, sind jedoch bisher an den erwähnten Stabilitätsproblemen gescheitert. Der Wunsch nach einer brauchbaren festen galenischen Form von Tilidin besteht insbesondere deshalb, weil nur über eine solche feste Formulierung eine steuerbare Retardierung des Tilidins realisierbar sein dürfte. Da Tilidin relativ kurz wirkt und eine gleichmäßige Schmerzbehandlung über einen längeren Zeitraum, etwa über Nacht, mit einer einzigen Wirkstoffdosis bei normaler Freigabe, wie sie flüssige Zubereitungen ganz allgemein bieten, problematisch ist, wäre die Bereitstellung einer solchen Retardform ein großer Fortschritt auf dem Gebiet der Analgetika, der insbesondere dort zum Tragen käme, wo ein gleichmäßig hoher Wirkstoffspiegel über lange Zeiträume gefordert wird, etwa bei der Bekämpfung chronischer und schwerer Schmerzzustände bei Krebserkrankungen oder bei Verbrennungen.

Aufgabe der vorliegenden Erfindung ist die Bereitstellung eines auch in festen und retardierbaren Arzneimittelformen, insbesondere Tabletten, Dragees oder Suppositorien stabilen Tilidin-Salzes und die Herstellung von insbesondere festen und retardierten Arzneimitteln mit diesem neuen Salz.

Es wurde nun überraschend gefunden, daß Tilidindihydrogenorthophosphat eine hervorragende Stabilität aufweist und sich als bisher einziges Salz für die Herstellung von festen Arzneimittelformen eignet, weil es in fester Form, das heißt in Verbindung mit festen Hilfsstoffen praktisch keine Zersetzung erleidet. Darüber hinaus hat das neue Salz überraschend pharmazeutisch-technologische Eigenschaften, die die Eigenschaften aller anderen vergleichbaren Tilidin-Salze, wie z.B. Tilidinhydrogensulfat oder Tilidinhydrogenfumarat übertreffen, so daß zum Beispiel ohne besondere Anforderungen an die Klimatisierung der Arbeitsräume und an den Korrosionsschutz der verwendeten Apparaturen und Werkzeuge, Granulate, Tabletten oder Suppositorien hergestellt werden können, die sowohl chemisch als auch physikalisch über einen sehr langen Zeitraum lagerungsstabil sind.

In der DE-PS 1 923 619 ist zwar pauschal erwähnt, daß basisch substituierte Cyclohexene mit einer Reihe von organischen und anorganischen Säuren, u.a. auch mit Phosphorsäure (Spalte 4, Zeile 19) Salze bilden können, die ggf. für eine Verarbeitung zu Arzneimitteln in Frage kommen, jedoch ist dort kein Phosphat beschrieben und insbesondere Tilidindihydrogenorthophosphat nicht erwähnt. Vor allem sind aber die obengenannten Stabilitätsprobleme speziell des Tilidins nicht beschrieben. Es gibt auch bisher in der Literatur keine Hinweise auf die Schwierigkeiten hinsichtlich der Langzeitstabilität dieses Wirkstoffs oder der technischen Probleme, wenn er zu festen Arzneimittelformen verarbeitet wird. Daher hatte der Fachmann bisher keine Veranlassung, sich über irgendwelche Auswahlkriterien Gedanken zu machen, insbesondere gab der bekannte Stand der Technik keinen Hinweis, daß sich gerade das Tilidindihydrogenorthophosphat zur Lösung der genannten Probleme besonders eignet.

Es hat sich außerdem gezeigt, daß die Herstellung des Phosphats kritisch ist und unter ganz bestimmten Bedingungen erfolgen muß, wenn man ein kristallines Produkt hoher Reinheit erhalten will. Zur Salzbildung geht man von etwa 80-90%iger, bevorzugt etwa 85-88%iger Orthophosphorsäure aus, die man in einer zur vollständigen Lösung ausreichenden Menge wasserhaltigen Isopropanols (Wassergehalt 4-10 Gew.%, bevorzugt 6 Gew.%) löst und diese Lösung bei einer Temperatur von 30-50°C, bevorzugt 40±5°C mit einer etwa 0,8-1,2 molaren, bevorzugt etwa 1 molaren Lösung von Tilidin-Base in Isopropanol des angegebenen Wassergehalts unter Rühren in etwa stöchiometrischen Mengen vereinigt und die entstehende Suspension unter Rühren langsam (über mehrere Stunden) abkühlt. Nach dem Waschen mit Isopropanol des oben angegebenen Wassergehalts erhält man so 99,5% reines Tilidinhydrogenorthophosphat in Ausbeuten über 90%. Die beiden Parameter Wassergehalt und Umsetzungstemperatur sind insofern sehr wichtig, als man bei Temperaturen unterhalb 35°C und abweichendem Wassergehalt mit Lösungsmittel verunreinigte und schlecht kristallisierende Produkte erhält, die sich nicht mehr vollständig trocknen lassen und Isopropanol hartnäckig festhalten.

Gegenstand der Erfindung ist daher das Tilidindihydrogenorthophosphat und dessen Herstellung. Ein weiterer Gegenstand der Erfindung sind eine feste und inbesondere eine retardierte feste galenische Arzneimittelzubereitung, bestehend aus üblichen festen Arzneimittelhilfsstoffen sowie gegebenenfalls Retardierungsmitteln, gekennzeichnet durch einen Gehalt an Tilidindihydrogenorthophosphat als Wirkstoff, sowie ein Verfahren zu deren Herstellung. Ein weiterer Gegenstand der Erfindung ist die Verwendung von Tilidindihydrogenorthophosphat bei der Herstellung fester insbesondere retardierter Arzneimittel formen.

Zur Herstellung retardierter fester Arzneimittelformen des Tilidins kommen alle üblichen Retardierungsverfahren in Frage, die die Stabilität des Wirkstoffs aufgrund ihrer Zusammensetzung nicht negativ beeinflussen. Besonders brauchbar ist eine gemäß EP-PS 0 043 254 durch ein Schmelzverfahren retardierte Tablette . Als Retardierungsmittel eignen sich aber ganz allgemein schwerlösliche Stoffe, wie z.B. lipide oder lipoide Substanzen wie Stearinsäure und insbesondere hydriertes Rizinusöl (Cutina HR) (DE-A 1 617 657, US-A 4 123 753) oder hydrophile Polymere, die als Quellstoffe die Freigabe des Wirkstoffs verzögern (J.Pharm.Sci. S.974 (1966). Zur gezielten Steuerung der Freigaberate kann auch das Verfahren gemäß EP-PS 0 068 446 eingesetzt werden, bei dem die Freigabegeschwindigkeit des Wirkstoffs aus einer mittels schwerlöslicher Substanzen retardierten Wirkstoffzubereitung durch die Viskosität eines zugegebenen hydrophilen Polymeren, wie z.B Methylcellulose oder Carboxymethylcellulose, eingestellt wird, ausgehend von der Erkenntnis, daß die Freigabegeschwindigkeit mit steigender Viskosität zunimmt.

Zum Nachweis der überraschenden überlegenheit des Dihydrogenorthophosphats wurden
a) Tilidinhydrochlorid-Semihydrat
b) Tilidinhydrogenfumarat
c) Tilidinhydrogensulfat und
d) Tilidindihydrogenorthophosphat
jeweils mit Naloxonhydrochlorid-Dihydrat und üblicherweise pharmazeutisch eingesetzten Hilfsstoffen verrieben. Das Naloxonhydrochlorid-Semihydrat ist ein wirksamer Morphinantagonist, den die im Handel befindlichen Tilidin-Zubereitungen enthalten. Bereits nach 28 Tagen Lagerung bei 60°C zeigten sich deutliche Unterschiede zwischen den vier Salzen: Während die Mischungen a) bis c) weitgehend verfärbt waren, zeigte keine der Mischungen von d) eine Farbänderung und damit Zersetzungserscheinungen.

In einem weiteren Versuch wurden drei verschiedene Salze des Tilidins jeweils mit Naloxonhydrochlorid-Dihydrat, hydriertem Rizinusöl, Laktose, Hydroxyethylcellulose, Stearinsäure, Tablettose und Magnesiumstearat zu Schmelzgranulat-Tabletten verarbeitet. Mit Tilidinhydrogenfumarat zeigte sich schon bei der Herstellung des Schmelzgranulates eine starke Grünfärbung. Die Tabletten mit Tilidinhydrochlorid-Semihydrat zeigten bereits nach zwei Tagen Lagerung in Braunglas bei 22°C orange-graue Verfärbungen. Die Tabletten mit Tilidindihydrogenorthophosphat hingegen zeigten unter denselben Bedingungen, auch nach sechsmonatiger Lagerung, keinerlei Verfärbungen.

Bei der Herstellung der Proben erwies sich das neue Dihydrogenorthophosphat auch hinsichtlich der Verarbeitungseigenschaften als außergewöhnlich gut.

Es ist nämlich in keiner Weise hygroskopisch, reagiert nicht mit metallischen Werkstoffen und ist inert gegenüber elektrostatischer Aufladung. Das Hydrochlorid-Semihydrat nimmt bei einer relativen Feuchte von 63% bereits beträchtliche Mengen Wasser auf und wirkt korrosiv an nicht besonders korrosionsgeschützten Werkzeugen. Das Schwefelsäuresalz nimmt bereits bei einer relativen Luftfeuchtigkeit von 58% Wasser auf und übertrifft das Hydrochlorid-Semihydrat in seinem aggressiven Korrosionsverhalten. Das Hydrogenfumarat hinwiederum neigt stark zu elektrostatischer Aufladung. Dies kann zu ungenauen Einwaagen und zur teilweisen Entmischung der Hilfs- und Wirkstoffe führen.

Das erfindungsgemäße Tilidindihydrogenorthophosphat wird wie folgt hergestellt:

### BEISPIEL 1

### Herstellung von Tilidindihydrogenorthophosphat

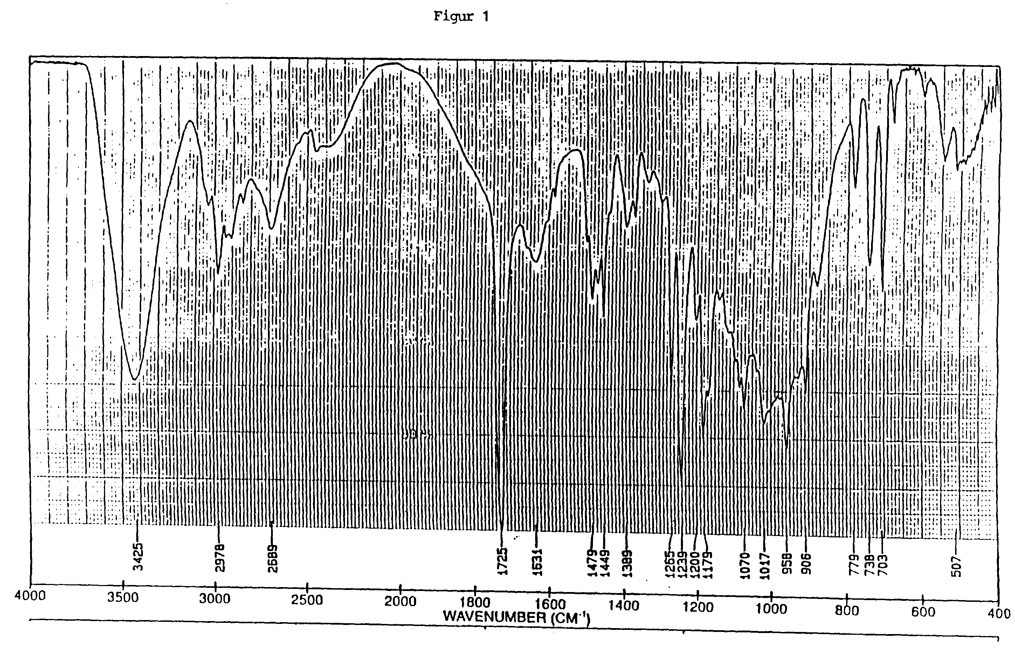

27,091 kg (99,10 mol) Tilidin-Base werden in 99 1 Isopropanol 94%ig (6% Wasser) unter Erwärmen auf etwa 40°C (±5°) gelöst. Unter Rühren wird eine Lösung aus 11,54 kg Orthophosphorsäure 85-88%ig (entsprechend 9,844 kg = 100,46 mol 100%ig) in 99 1 Isopropanol 94%ig im Verlauf von 3 Stunden bei 40°C zugegeben. Zur besseren Kristallisation werden ständig Impfkristalle zugesetzt. Die Suspension wird unter Rühren langsam über Nacht auf Raumtemperatur abgekühlt. Die Kristalle werden abzentrifugiert und mit 2 mal 10 1 Isopropanol 94 %ig gewaschen. Das weiße Salz wird bei 50-60°C getrocknet. Ausbeute: 33,89 kg (92,09% d.Th.) Das Salz weist eine analytische Reinheit (HPLC) von 99,5% auf. Schmp. 137,0°C. Molmasse: 371,37. Das IR-Spektrum ist in Figur 1 wiedergegeben.

### BEISPIEL 2

### Retardtablette enthaltend 120 mg Tilidindihydrogenorthophosphat

960 g Tilidindihydrogenorthophosphat, hergestellt nach Beispiel 1, werden mit 70,4 g Naloxonhydrochlorid-dihydrat, 740 g Lactose, und 700 g hydriertem Rizinusöl vorgemischt und dann langsam und unter fortgesetzter Durchmischung bis zu einer Produkttemperatur von 83°C aufgeheizt. Die entstandene Schmelzmasse wird ausgetragen und über ein Sieb von 2,5 mm passiert. Nach Abkühlung auf Raumtemperatur wird durch ein weiteres Sieb (1 mm Maschenweite) passiert. Anschließend werden 273,6 g Ammonio Methacrylat Copolymer, 32 g Magnesiumstearat und 24 g Siliciumdioxid gesiebt und gleichmäßig unter das Granulat gemischt. Die Mischung wird dann mit einer Exzenterpresse zu runden, leicht gewölbten Tabletten von 10 mm Durchmesser, 11 mm Wölbungsradius und 350 mg Sollgewicht verpreßt. Die erhaltenen Tabletten haben folgende Zusammensetzung:

| | |
|---|---|
| Tilidindihydrogenorthophosphat | 120,0 mg |
| Naloxonhydrochlorid-dihydrat USP | 8,8 mg |
| Laktose | 92,5 mg |
| Hydriertes Ricinusöl | 87,5 mg |
| Ammonio Methacrylate Copolymer | 34,2 mg |
| Magnesiumstearat | 4,0 mg |
| Siliciumdioxid | 3,0 mg |

Für die erhaltenen Tabletten wurden folgende Produkteigenschaften ermittelt:
Masse Mittelwert: 351 mg, Minimum 340 mg, Maximum 362 mg, Sᵣₑ₁ 1,8%
Bruchfestigkeit: Mittelwert 82 N, Minimum 64 N, Maximum 90 N, Sᵣₑ₁ 8,4%
Friabilität: 0,23%
Zerfall: über 3 Stunden.

### BEISPIEL 3

### Suppositorien enthaltend 59.93 mgTilidindihydrogenorthophosphat

9700 g Hartfett einer Hydroxylzahl von 40-50 (Witepsol W 35®), bzw. alternativ Hartfett einer Hydroxylzahl von bis zu 2 (Massa Estarinum R 299), wird in einem beheizbaren Kessel mit Rührwerk bei 45°C vollständig klar aufgeschmolzen. Nach dem Abkühlen auf 36 bis 38°C werden 300g Tilidindihydrogenorthophosphat mit Hilfe eines Ultra-Turrax® Mischers in der geschmolzenen Fettgrundlage dispergiert. Die Produkttemperatur wird dabei gegebenenfalls unter Kühlung des Kesselmantels auf 36 bis 38°C gehalten. Die gießfertige Schmelze wird dann in einer Form- Füll- und Verschließmaschine in Suppositorienblister aus Dreifachverbundfolie ausgegossen. Die Gießtemperatur an der Dosierstation wird bei 37°C gehalten. Die Kesseltemperatur beträgt während des Ausgießens 37°C. Die Dosiermenge pro Füllstation beträgt 2,0 g ± 5%. Die Zäpfchen werden während des Transportes durch einen Kühltunnel, in dem eine Temperatur von 20 bis 22°C herrscht, abgekühlt und dadurch verfestigt. Die Blister werden anschließend heißversiegelt und ausgestanzt. Pro Stunde können so bis zu 9000 Zäpfchen gefertigt werden. Der vorliegende Ansatz ergab 4850 Zäpfchen enthaltend 59,93 mg Tilidindihydrogenorthophosphat (entsprechend 50 mg Tilidinhydrochlorid).

## Patentansprüche

1. Tilidindihydrogenorthophosphat

2. Verfahren zur Herstellung von Tilidindihydrogenorthophosphat, dadurch gekennzeichnet, daß man Tilidin-Base in an sich bekannter Weise mit Orthophosphorsäure umsetzt und das Salz isoliert.

3. Verfahren nach Anspruch 2, dadurch gekennzeichnet, daß man die Umsetzung mit 80-90%iger Phosphorsäure in Isopropanol mit einem Wassergehalt von 4-10 Gew.% bei einer Temperatur von 30-50°C, bevorzugt 40±5°C. durchführt.

4. Arzneimittel, das neben üblichen Hilfsstoffen als Wirkstoff ein Tilidin-Salz enthält, dadurch gekennzeichnet, daß das Tilidin-Salz als Dihydrogenorthophosphat vorliegt.

5. Arzneimittel nach Anspruch 4, dadurch gekennzeichnet, daß es in fester Form vorliegt.

6. Arzneimittel nach Anspruch 5, dadurch gekennzeichnet, daß es zusätzlich ein Retardierungsmittel enthält.

7. Arzneimittel gemäß Anspruch 5 oder 6 in Form von Tabletten, Dragees, Kapseln, Suppositorien oder Granulat.

8. Verwendung von Tilidindihydrogenorthophosphat zur Herstellung von festen Arzneimittelformen.

9. Verwendung von Tilidindihydrogenorthophosphat gemäß Anspruch 8 zur Herstellung von retardierten Arzneimittelformen.

## Claims

1. Tilidine dihydrogen orthophosphate.

2. Process for the preparation of tilidine dihydrogen orthophosphate, wherein tilidine base is reacted in known manner with orthophosphoric acid and the salt then isolated.

3. Process according to claim 2, characterised in that the reaction with 80-90% phosphoric acid in isopropanol with a water content of 4-10% by weight is carried out at a temperature of 30-50°C, preferred 40±5°C.

4. Pharmaceutical composition which, beside conventional adjuvants, contains as active material a tilidine salt, wherein the tilidine salt is present as the dihydrogen orthophosphate.

5. Pharmaceutical composition according to claim 4, wherein it is present in solid form.

6. Pharmaceutical composition according to claim 5, wherein it additionally contains a retarding agent.

7. Pharmaceutical composition according to claim 5 or 6 whenever in the form of a tablet, coated tablet, capsule, suppository or granulate.

8. The use of tilidine dihydrogen orthophosphate for the production of solid pharmaceutical compositions.

9. The use of tilidine dihydrogen orthophosphate according to claim 8 for the production of retarded pharmaceutical compositions.

## Revendications

1. Dihydrogéno-orthophosphate de tilidine.

2. Procédé de préparation de dihydrogéno-orthophosphate de tilidine, caractérisé en ce qu'on fait réagir de la tilidine base, de façon connue avec de l'acide orthophosphorique et on isole le sel obtenu.

3. Procédé selon la revendication 2, caractérisé en ce que la réaction est mise en oeuvre en présence d'une solution de 80-90% d'acide phosphorique dans de l'isopropanol présentant une teneur en eau de 4 à 10% en poids, à une température comprise entre 30 et 50%, de préférence de 40±5°C.

4. Médicament contenant à titre de principe actif, outre les adjuvants convenables, un sel de tilidine, caractérisé en ce que le sel de tilidine est présent sous forme de dihydrogéno-orthophosphate.

5. Médicament selon la revendication 4, caractérisé en ce qu'il est sous forme solide.

6. Médicament selon la revendication 4, caractérisé en ce qu'il contient en outre un agent retard.

7. médicament selon la revendication 5 ou 6, sous forme de comprimés, dragées, gélules, suppositoires ou granulés.

8. Utilisation de dihydrogéno-orthophosphate de tilidine pour la fabrication de médicaments sous forme solide.

9. Utilisation de dihydrogéno-orthophosphate de tilidine selon la revendication 8, pour la fabrication de médicaments à libération retardée.
